# EUROPEAN PATENT SPECIFICATION

(11) **EP 4 373 441 B1**
(45) Date of publication and mention of the grant of the patent: **23.09.2026**
(21) Application number: 22747386.5
(22) Date of filing: 13.07.2022
(51) Int. Cl.: A61F 2/64

(54) **A PROSTHETIC JOINT AND PROSTHETIC LIMB COMPRISING THE SAME**
PROTHESENGELENK UND PROTHESENGLIED DAMIT
ARTICULATION PROTHÉTIQUE ET MEMBRE ARTIFICIEL LA COMPRENANT

(30) Priority: 22.07.2021 GR 20210100497; 29.07.2021 GB 202110928
(43) Date of publication of application: 29.05.2024
(73) Proprietor: Imperial College Innovations Ltd, London SW7 2AZ (GB)
(72) Inventor: VARDAKASTANI, Vasiliki, London SW7 2AZ (GB); MILANDRI, Giovanni Sergio, London SW7 2AZ (GB); FAVIER, Clément David, London SW7 2AZ (GB); HENSON, David Peter, London SW7 2AZ (GB)
(74) Representative: HGF
(86) International application number: PCT/GB2022/051812
(87) International publication number: WO 2023/002159

(56) References cited:
- EP-B1- 2 432 430
- US-A1- 2014 188 252
- US-A1- 2015 359 643

## Description

This invention relates to a prosthetic joint, such as a prosthetic knee joint or a prosthetic elbow joint, and a prosthetic limb including a prosthetic joint.

### BACKGROUND

Existing prosthetic knee joints only provide a limited range of motion compared to the anatomical joint they aim to replace. This is because the conventional linkage mechanism used to provide a polycentric joint causes interference between the components of the prosthetic limb and socket at large angles of flexion. This is because the traditional arrangement of linkages are to provide a single posterior linkage with a pair of anterior linkages that are disposed either side of the knee. Examples of such polycentric knee joint prostheses are disclosed in US 2014/188252 A1, US 2015/359643 A1, and EP 2432430 B1. As such, in certain communities and/or in young children, where kneeling, sitting cross-legged and/or squatting are typical activities of daily living, prior art devices are unable to enable above knee (AK) and through-knee (TK) amputees to fully participate in daily life.

When amputation is necessary, it is also desirable to maintain as much of the original limb as possible, and in the case of through-knee amputees, the entire femur can be preserved. However, through-knee amputees suffer from the problem known as 'thigh lengthening', where the thigh of the amputated limb is longer than the thigh of the remaining leg (in the case of unilateral amputees) due to the need to incorporate a socket and other connections between the socket and the prosthetic knee. The material of the distal socket and the proximal part of the prosthetic joint effectively extend the length of the thigh, creating a limb length discrepancy which in turn has negative consequences from a biomechanical and cosmetic and social perspective.

A further problem with existing devices is the need to use specialist tools and/or a trained technician when servicing prosthetic limbs. Typically, an amputee will need to bring their device to a professional to service as they will not have the specialist tools required, which may require considerable travel. This reliance on specialist tools and/or a technician renders amputees unable to adjust their own devices as they need. Consequentially, patients will often have to endure a poorly-fitted device as they go about their daily activities, which can result in poor engagement between the amputee and their prosthetic.

The present invention seeks to address at least some of these issues.

### BRIEF SUMMARY OF THE DISCLOSURE

The invention is solely defined in the claims. Any subject-matter that is disclosed herein, but which is not defined in the claims, does not form part of the invention.

Viewed from a first aspect, the present invention provides a prosthetic joint comprising: a proximal part having a proximal side, a distal side opposed to the proximal side, and a mounting point on the proximal side configured to connect to a proximal prosthetic limb part, a distal part configured to connect to a distal prosthetic limb part, a polycentric linkage mechanism connected to the proximal and distal parts and arranged to rotate the distal part from an extended position, where the distal part is disposed on the distal side of the proximal part, to a flexed position, where at least a portion of the distal part is disposed on the proximal side of the proximal part. The distal part is arranged to rotate relative to the proximal part about an instantaneous axis of rotation, the axis of rotation defining a medial-lateral axis of the proximal part. The linkage mechanism comprises a first linkage and a further pair of linkages, each of which are pivotally connected to the proximal and distal parts via respective connection points. The position of the instantaneous axis of rotation is determined by the location of the connection points of the first linkage and the further pair of linkages. The connection points of the first linkage are spaced from a mid-plane of the proximal part by a smaller distance along the medial-lateral axis than the connection points of the pair of linkages. The distal part is spaced from the proximal part in the direction of flexion as the distal part is rotated between the extended position and the flexed position. In the extended position, the connection points of the first linkage are spaced from the mounting point by a smaller distance in the direction of flexion than the connection points of the pair of linkages.

By inverting the traditional linkage arrangement in polycentric joints, the present linkage mechanism allows for greater angles of flexion of the joint compared to prior art devices. This is possible as the first linkage is able to pass through the space created between the pair of linkages spaced in the direction of flexion. In the case of a knee joint, the anterior linkage is able to pass between the posterior linkages in deep flexion. In the case of an elbow joint, the posterior linkage is able to pass between the anterior linkages in deep flexion. As such, the range of motion of the present prosthetic joint is comparable to that of the anatomical joint it is replacing, such as the knee or elbow, thus enabling a greater range of activities such as kneeling and squatting.

In the extended position, the first linkage may form a first angle relative to a longitudinal axis of the proximal part and the pair of linkages may form a second angle relative to the longitudinal axis of the proximal part. The second angle may be smaller than the first angle. In the extended position, the pair of linkages may form an angle of between 0 and 15 degrees relative to a longitudinal axis of the proximal part. In the extended position, the first linkage may form an angle of between 5 and 30 degrees relative to a longitudinal axis of the proximal part. In the extended position, the first linkage and the pair of linkages may be oriented in the direction of flexion.

The connection points of the first linkage and the pair of linkages may be disposed on the same side of the mounting point. For example the anterior side or the posterior side of the proximal part when in the extended position.

The direction of flexion may be a posterior direction or an anterior direction relative to the proximal part.

The distal part may comprise a channel for receiving a portion of the distal prosthetic limb part, and a clamp for releasably securing the portion of the distal prosthetic limb part in the channel. The position of the distal prosthetic limb part within the distal part may therefore be adjusted to provide a longer or shorter leg length, such that a single device can account for growth in a child, or the same device can be configured for people of different heights. For example, 40mm or more adjustability may be provided within the same device. Additionally or alternatively, the distal prosthetic limb part may be adjusted about a longitudinal axis of the distal part, e.g. to provide greater or lesser internal-external foot rotation angle. The longitudinal axis of the distal pylon part may be a superior-inferior axis of the distal part when in the extended position.

The clamp may comprise a body formed as an open-ended loop with a pair of ends spaced from one another, a pin extending through the pair of ends and fixed to one of the pair of ends, and a lever rotatably connected to the pin. The lever may be eccentrically mounted to the pin to provide a camming surface on the lever. When rotated to a closed position, the camming surface may be arranged to abut one of the ends of the body so as to deform the body to clamp the distal prosthetic limb part to the distal part. When rotated to an open position, the camming surface may be arranged to release the body from the distal prosthetic limb part so as to allow the distal prosthetic limb part to move relative to the distal part. This advantageously provides the user of the prosthetic joint the ability to easily adjust their distal prosthetic limb part without additional tools.

The prosthetic joint may further comprise an extensor system configured to apply a torque to rotate the distal part to the extended position. This advantageously provides greater assistance to the user during walking. The amount of torque may vary according to the angle of rotation of the distal part, so as to provide greater support for a variety of walking speeds.

The extensor system may be arranged to apply a peak torque when the distal part is rotated between 20 and 40 degrees from the extended position. Adjusting the joint flexion angle at which peak torque is applied provides greater adaptability of the prosthetic joint for different individuals, who may walk at different speeds.

The extensor system may be biased to rotate the distal part to the extended position across a range of motion of the linkage mechanism. For example, the torque may only rotate the distal part to the extended position. This avoids the "pocket knife" phenomenon where prior art devices will "flip" to cause the joint to flex beyond a certain angle of flexion.

The extensor system may comprise a tensile element connected to a resiliently deformable element and the proximal part. As the distal part rotates from the extended position to the flexed position, the resiliently deformable element may be arranged to deform so as to exert the torque. The tensile element is preferably a cable, such as a steel cable. The resiliently deformable element is preferably a compression spring.

The resiliently deformable member may be disposed within a tubular element of the distal part. At least a portion of the tensile element may extend through the tubular element. The tubular element may be part of the distal prosthetic limb part (e.g. the pylon of a lower limb) or may be distinct from the distal prosthetic limb part.

The extensor system may comprise a locking member arranged to selectively lock the tensile element to prevent further rotation of the distal part towards the flexed position. The locking member may be a sliding plate. The locking member may include an opening through which the tensile element having a bulbous portion can pass. The opening preferably has at least two portions. In an unlocked position, the bulbous portion may pass freely through a first portion of the opening. In a locked position, a second portion of the opening, smaller than the first portion, may prevent the bulbous portion from passing therethrough. Alternatively or additionally, the locking member may grip or otherwise engage the tensile element to prevent its movement.

The locking member may be operable by a hand of a user. This advantageously allows a user to easily lock the knee in the extended position to provide stability when standing, and to unlock the knee when they wish to flex the knee.

The torque of the extensor system may be adjustable by a hand of a user. This advantageously allows for tool-free adjustment of the joint.

The mounting point may be configured to connect to an adapter, such as a pyramid adapter, or directly to a socket. Thus, patients with osseointegration are able to use the prosthetic joint by using an adapter, such as a pyramid adapter.

The proximal part may comprise a recess for receiving a portion of the proximal prosthetic limb part. The recess may include a spherical portion which allows for a direct connection with the International Committee of the Red Cross (ICRC) system. The proximal part may comprise at least one gripping member for securing the proximal prosthetic limb part thereto.

The linkage mechanism may be arranged to provide a range of motion of at least 160 degrees between the extended position and the flexed position.

The proximal part may comprise a proximal end and a distal end. The distance between the proximal and distal ends may be as little as to 40mmpreferably as little as 30mm. This advantageously provides a reduced thigh length compared to prior art devices.

When the distal part is rotated by 90 degrees or more from the extended position, the linkage mechanism may be spaced from the proximal part in the direction of flexion. When the distal part is rotated by 90 degrees or more from the extended position, the proximal part may provide a load path from the mounting point to an external surface abutting the distal side of the proximal part. This allows a user to kneel directly on the ground without having to transmit their weight through the linkage mechanism. This improves the longevity of the prosthetic joint. The distal part may comprise a support for supporting the proximal prosthetic limb part when the joint is rotated to maximum flexion, so as to provide a load path between the proximal prosthetic limb part and an external surface via the distal part. The external surface may be a ground surface. Thus, the transition from kneeling upright with the proximal part being in contact with the ground, to sitting on the heels with the distal part in contact with the ground, is smooth as the linkage mechanism is moved out of the path between the socket and the ground. This is similar to an anatomical joint and provides improved ergonomics of the joint.

The prosthetic joint may be configured as a knee joint or an elbow joint.

Viewed from a further independent aspect there is also provided a prosthetic limb comprising: a prosthetic joint according to any preceding claim, a proximal prosthetic limb part connected to the proximal part of the prosthetic joint, and a distal prosthetic limb part connected to the distal part of the prosthetic joint.

Viewed from a further aspect, there is provided a prosthetic knee joint comprising: a proximal part having a proximal side, a distal side opposed to the proximal side, and a mounting point on the proximal side configured to connect to a proximal prosthetic limb part, a distal part configured to connect to a distal prosthetic limb part, a linkage mechanism connected to the proximal and distal parts and arranged to rotate the distal part from an extended position, where the distal part is disposed on the distal side of the proximal part, to a flexed position, where at least a portion of the distal part is disposed on the proximal side of the proximal part. The distal part is arranged to rotate relative to the proximal part about a polycentric axis of rotation, the axis of rotation defining a medial-lateral axis of the proximal part. The linkage mechanism comprises a first linkage and a further pair of linkages, each of which are pivotally connected to the proximal and distal parts via respective connection points. The position of the instantaneous axis of rotation is determined by the location of the connection points of the first linkage and the further pair of linkages. The connection points of the first linkage are spaced from a mid-plane of the proximal part by a smaller distance along the medial-lateral axis than the connection points of the pair of linkages. The distal part is spaced from the proximal part in a posterior direction relative to the proximal part as the distal part is rotated between the extended position and the flexed position. In the extended position, the connection points of the first linkage are spaced from the mounting point by a smaller distance in the posterior direction than the connection points of the pair of linkages.

### BRIEF DESCRIPTION OF THE DRAWINGS

Embodiments of the invention are further described hereinafter with reference to the accompanying drawings, in which:
Figures 1A and 1B illustrate perspective views of an exemplary prosthetic knee joint;
Figure 2 is a schematic representation of the joint of Figure 1;
Figure 3 illustrates a cross-sectional view of the joint of Figure 1 with the outer parts made transparent;
Figure 4 illustrates an exemplary clamp for clamping a distal prosthetic limb part to the prosthetic joint;
Figures 5A to 5D illustrate cross-sectional views of an exemplary prosthetic limb rotating from an extended position to a flexed position;
Figures 6A and 6B illustrate cross-sectional views of an exemplary extensor system with the outer parts made transparent with the joint of Figure 1 in an extended and flexed position;
Figure 7 illustrates a rear view of the joint of Figure 1, with some parts omitted for clarity;
Figures 8A and 8B illustrate an exemplary locking member in locked and unlocked configurations, with some parts omitted for clarity;
Figures 9 to 11 illustrate the change in different parameters of the extensor system with knee flexion;
Figures 12A and 12B respectively illustrate perspective and side views of an alternative prosthetic knee joint;
Figure 13 is a schematic representation of the joint of Figures 12A and 12B;
Figures 14A and 14B illustrate cross-sectional views of an alternative extensor system with the outer parts made transparent in an extended and flexed position;
Figures 15A and 15B illustrate an alternative locking member in locked and unlocked configurations.

### DETAILED DESCRIPTION

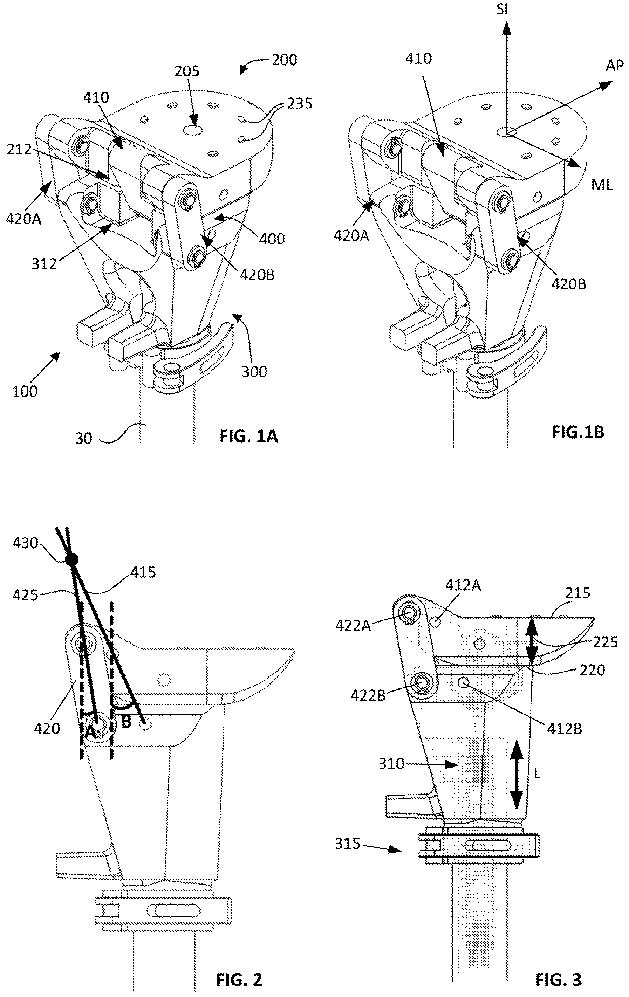

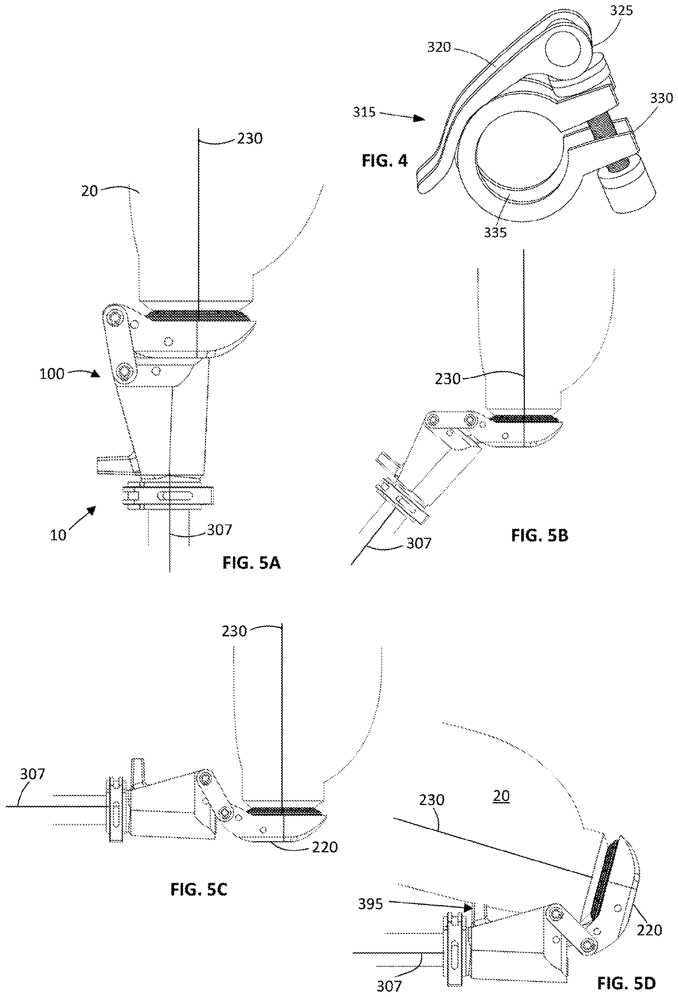

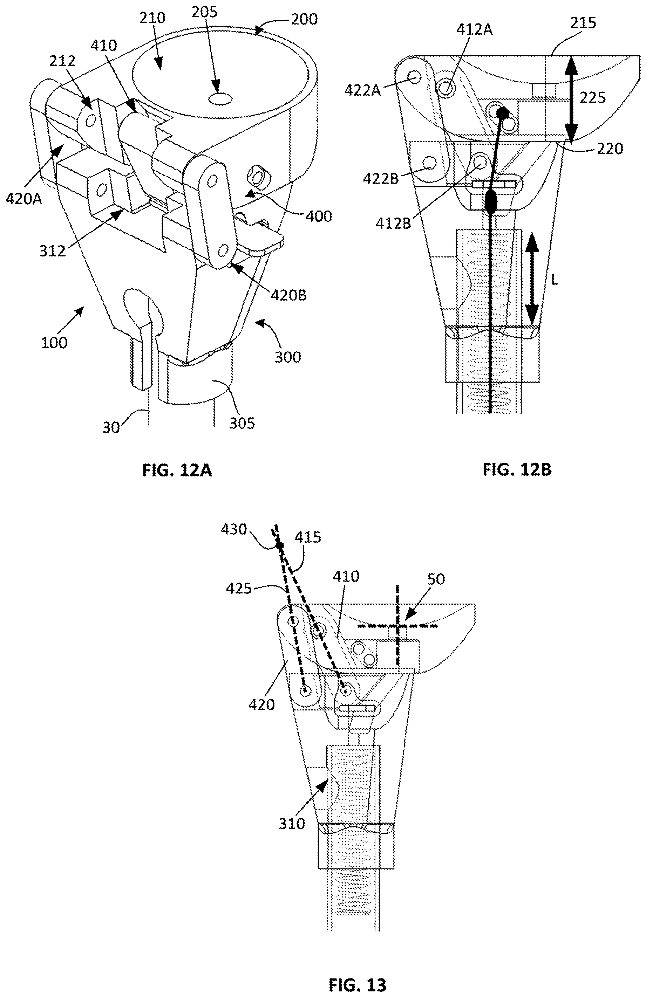

The prosthetic knee joint 100 illustrated in Figure 1A includes a proximal part 200, a distal part 300 and a linkage mechanism 400. The proximal part 200 is typically secured to a socket 20 (see Figure 5) either directly or indirectly. Where an indirect connection is needed, an adapter, such as a pyramid adapter can be used to provide compatibility between a mounting point at the distal end 50 of the socket 20 and the mounting point 205 of the proximal part 200. This connection enables the prosthetic limb to be secured to the socket 20, so that there is minimal relative movement between the residual limb and the proximal part 200. Where the distal end of the socket 20 has a known shape, such as that used in the low-cost International Committee for the Red Cross (ICRC) system, a recess 210 having a corresponding profile (such as a spherical profile in the case of the ICRC system) can be formed in a proximal side 215 of the proximal part 200 to enable a direct connection between a mounting point 205 of the proximal part 200 and the distal end 50 of the socket 20 (such as shown in Figures 12A, 12B and 13). This is particularly useful in through-knee amputees, as a direct connection avoids the need for additional material on the proximal side of the prosthetic joint 100 which would compound the thigh lengthening problem. For above-knee amputees, the recess 210 may be omitted and a pylon (not shown) may be used to connect the socket 20 to the mounting point 205. As shown in Figures 1A and 1B, a proximal side of the proximal part 200 may be a flat surface having one or more teeth 235 configured to grip other ICRC configurations. It would be apparent the teeth 235 may be present in a recessed proximal part 200.

When the prosthetic limb is assembled, the distal part 300 is connected to a pylon 30, which may in turn be connected to a prosthetic foot (not shown). A portion of the pylon 30 is secured within a channel 310 formed in the distal part 300 (see Figure 3) using a releasable clamp 315. As the pylon 30 can be gradually removed from the channel 310 by sliding as the user requires, this provides a simple way to provide a limb with an adjustable length, which is particularly beneficial to children. The distal part 300 illustrated in Figure 3 provides approximately 40mm of adjustability "L", however, it would be apparent this was merely exemplary and ranges of adjustability less than 40mm or greater than 40mm may be included depending on the requirements of the prosthetic joint. The clamp 315 has a body formed as an open-ended loop with ends 330 through which a pin extends and a lever 320 rotatably connected to the pin. The pin is connected to a lever 320 at one end and a nut at the other end. The lever 320 is eccentrically mounted to the pin to provide a camming surface 325 on the lever 320 that is arranged to abut the end of the body so that an inner surface 335 of the body will clamp a clamping portion 305 of the distal part 300 when closed, and is arranged to release the end 305 when open. A "quick release" clamp is suitable for use as clamp 315 as shown in Figure 4.

Figures 2 and 3 illustrate cross-sectional views of the prosthetic joint 100 of Figures 1A and 1B and show an exemplary linkage mechanism 400 including one anterior linkage 410 and a pair of posterior linkages 420A, 420B. The terms "anterior" and "posterior" are taken from the frame of reference of the proximal part 200 along the "AP" (anterior-posterior) axis (see Figure 1B). The origin is shown coincident with the mounting point 205 in the Figures, but it would be apparent that this was not essential, as the mounting point may be positioned elsewhere, or multiple mounting points may be used. The linkages 410, 420, in combination with the proximal 200 and distal 400 parts provide a 4-bar linkage system which enables the distal part 300 (and the connected pylon and prosthetic foot) to be rotated relative to the proximal part, as shown in Figures 5A to 5D. As indicated in Figure 3, the linkages 410, 420 are pivotally connected to the proximal part 200 via connection points 412A, 422A, and to the distal part 300 via connection points 412B, 422B respectively. By positioning the connection points 412, 422 posterior to the distal end 50 of the socket 20, the linkage mechanism 400 allows full range of motion without interference between the parts of the prosthetic joint and the socket. Furthermore, the connection points 422A, 422B of the posterior linkages 420 are spaced from the mounting point 205 by a greater distance, taken along the AP axis, than the distance between the connection points 412A, 412B of the anterior linkage 410. Thus, the posterior linkages 420 can be considered to be positioned "posteriorly" to the anterior linkage. The mounting point 205 is preferably spaced anteriorly to the linkage mechanism 400 by at least 40mm to accommodate the ICRC system. That is to say the distance between the connection points 412A, 412B of the anterior linkage 410 are at least 40mm from the mounting point 205 in the anterior direction.

The posterior linkages 420 are also spaced from a mid-plane coincident with the "SI" (superior-inferior) and "AP" axes as shown in Figure 1B. The distance between the posterior linkages 420 and the mid-plane, taken on the ML (medial-lateral) axis, is larger than the distance between the anterior linkage 410 and the mid-plane. While the anterior linkage 410 intersects the mid-plane in Figure 1B, it would be apparent this was not essential, and an anterior linkage may be spaced from the mid-plane. Similarly, while a single anterior linkage 410 is shown, it would be apparent this was not essential, and the same functionality of the anterior linkage 410 described herein may be provided by two or more linkages.

The instantaneous axis of rotation 430 is the axis at any given angle of knee flexion about which the proximal 200 and distal 300 parts rotate relative to one another. The axis of rotation 430 can be derived from the intersection of the longitudinal axis 415 of the anterior linkage 410 and the longitudinal axis 425 of the posterior linkages 420 as illustrated in Figure 2. Thus, by orienting the anterior 410 and posterior 420 linkages in a posterior direction (i.e. the direction of flexion in the knee) as shown in Figures 2 and 3, i.e. the direction from connection point 422B to 422A, and 412B to 412A is posterior, the instantaneous axis of rotation 430 is positioned above and behind the mounting point 205 of the proximal part 200 during the initial movement from the extended position. This helps achieve a full range of motion without interference between the distal part 300 and the socket 20 and/or the proximal part 200. This is in contrast to prior art devices where the posterior linkage is oriented in an anterior direction (i.e. in opposition to the direction of flexion in the knee), while the anterior linkage is oriented in the posterior direction.

Prior art devices have a relatively parallel arrangement of linkages which locates the instantaneous axis of rotation 430 further above the knee, consequently requiring a larger radius of rotation of the distal part 300 relative to the proximal part 200. Contrary to this, the anterior linkage 410 and posterior linkages 420 of the present joint 100 are angled (when viewed from a mid-plane of the proximal part 200) relative to one another by a greater amount in order to position the axis of rotation closer to the proximal part initially, further reducing the risk of interference between the components of the prosthetic limb at maximum flexion. An angle between the anterior linkage 410 and the SI axis (angle "B" in Figure 2) of between 5 and 30 degrees in the extended position has been found to be particularly effective. An angle between the posterior linkages and the SI axis (angle "A" in Figure 2) of between 0 and 15 degrees in the extended position has been found to be sufficient to achieve the desired range of motion. Arranging the linkages 410, 420 such that the anterior linkage 410 has a smaller angle relative to the longitudinal axis than the posterior linkages 420 in the extended position is preferable.

The present linkage mechanism 300 also enables a more compact form of proximal part 200 to be used, with the height 225 of the proximal part 200 being 40mm. The height 225 of the proximal part 200 is taken in the longitudinal direction 230 between the proximal 215 and distal sides 220 of the proximal part 200 along the SI axis shown in Figure 1B.

Figures 5A to 5D illustrate cross-sectional views of an exemplary prosthetic limb 10 rotating from an extended position (Figure 5A) to a fully flexed position (Figure 5D). The joint angle can be taken as the relative rotation of the longitudinal axis 307 of the distal part 300 relative to the longitudinal axis 230 of the proximal part 200. The longitudinal axis 307 of the distal part 300 is parallel to an SI axis of the distal part 300 in the extended position. Figures 5A to 5D illustrate the prosthetic limb 10 at zero degrees of flexion (i.e. with the knee in full extension), 40 degrees of flexion, 90 degrees of flexion and 160 degrees of flexion. As shown in Figure 5D, at flexion angles between 160 and 165 degrees there will normally be interference between the distal part 300 and the socket 20 in through-knee amputees. By locating the posterior 420 linkages further apart from the mid-plane than the anterior linkage 410 and also more posteriorly than the anterior linkage 410, the proximal 200 and distal parts 300 can include respective notches 212, 312 in the posterior aspects of the proximal 200 and distal 300 parts respectively. This enables unimpeded movement of the anterior linkage 410 and the socket 20 throughout the complete range of motion of the prosthetic joint, which in isolation of a socket 20, is up to 180 degrees. While notches 212, 312 are shown, it would be apparent this was merely one way of shaping the proximal 200 and distal parts 300 so as to avoid interference between the components with large angles of knee flexion (e.g. greater than 140 degrees). The proximal part 200 also includes a compression contact surface (not shown) on the distal side 220 so that an amputee can kneel on their prosthetic knee without loading the linkage mechanism 300, which could otherwise damage the linkage mechanism. This is achieved by the linkage mechanism 300 moving to a posterior position relative to the proximal part 200 at larger angles of knee flexion (as shown in Figures 5C and 5D). By spacing the linkage mechanism 300 posteriorly relatively to the proximal part 200, the amputee can transition between kneeling on the ground (where the proximal part 200 contacts the ground) and sitting on their heels (where the distal part 300 may also be pressed into the ground against the socket 20) without risk of damaging the linkages 410, 420. As the distal part 300 includes a pair of supports 395 for supporting the socket 20 when the knee is in full flexion, a load path is provided between the socket 20 and the distal part 300 via the supports 395 such that the load does not have to pass through the linkage mechanism 400. While a pair of supports 395 are shown, it would be apparent this was not essential, and that a single support may be used to support the socket 20 in full flexion.

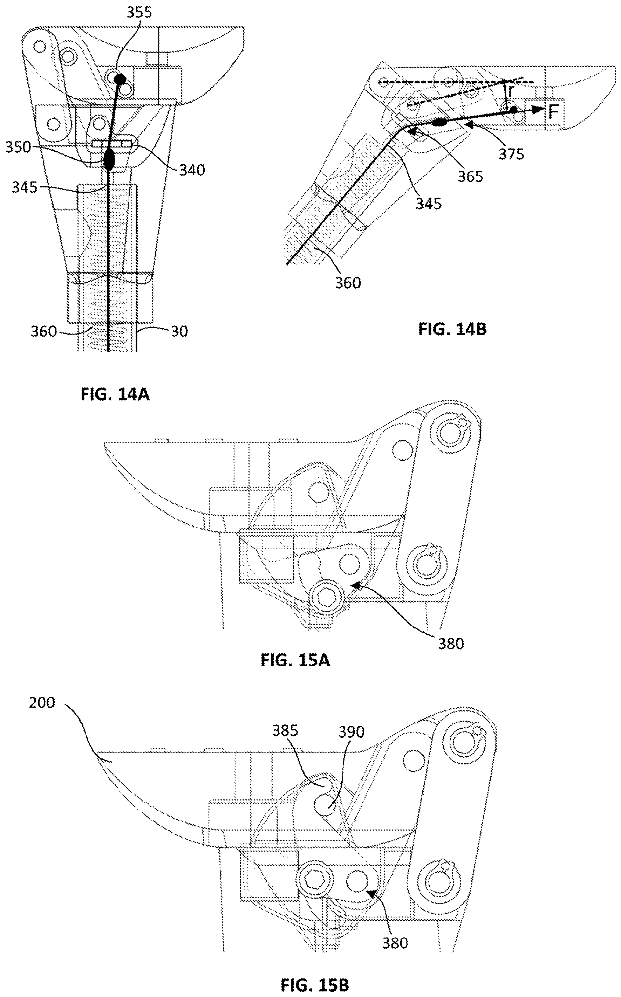

Figures 14A and 14B illustrate cross-sectional views of an exemplary extensor system with the outer parts made transparent. The extensor system includes a steel cable 345 and a compression spring 360. The steel cable 345 is anchored to the proximal part 200 by an anchor 355 at one end, and is connected to the compression spring 360 at the other end. The compression spring 360 is secured to the distal part 300 via the pylon 30. In the illustrated system, the compression spring 360 is disposed within the pylon 30 and the steel cable 345 enters the top of the pylon 30 and is connected to the compression spring 360 within the pylon. However, it would be apparent this was not essential, and the compression spring 360 may be secured to the distal part 300 directly. As the knee flexes (as shown in Figure 14B), the increased distance between the anchor 355 and the connection with the compression spring 360 will draw the steel cable 345 out of the pylon, which will in turn compress the compression spring 360 and generate a tensile force "F" within the cable 345. As this force F is applied at a distance normal to the axis of rotation 430, this will exert a torque about the instantaneous axis of rotation 340 which will act to urge the knee to rotate to the extended position. A shoulder 365 formed on the distal part 300 may act as a via point over which the steel cable 345 will pass. The shoulder 365 and the anchor 355 can define a force vector F of the steel cable 345 which, in combination with the instantaneous centre of rotation 430, can be used to derive the extensor torque applied to the knee as explained below. At very large angles of knee flexion, the cable 345 will contact the proximal part 200, which will further adjust the direction of the force vector **F,** and therefore maintain the moment arm "r" in a similar manner to the anatomical patellar.

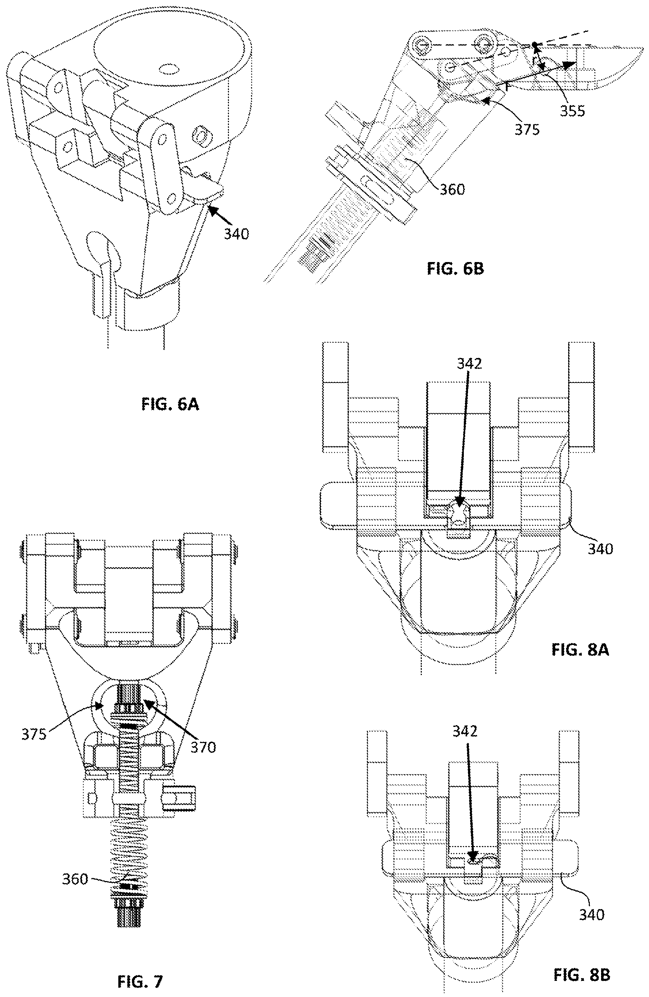

The distal part 300 includes a locking system to lock the knee joint in extension to provide greater stability for the amputee when standing. As shown in Figures 8A and 8B, the locking system includes a locking plate 340 for securing the steel cable 345 in position and a bulbous portion 350 (see also Figures 14A and 14B) which passes through an opening 342 formed in the locking plate 340. The opening 342 has two widths. A first width is larger than the cross-sectional width of the bulbous portion 350 (see also Figures 14A and 14B), and a second width is smaller than the cross-sectional width of the bulbous portion 350. As the locking plate 340 is slidably mounted to the distal part 300, a user can slide the locking plate 340 between an unlocked position (Figure 8A) wherein the bulbous portion 350 can pass through the wider part of the opening 342, and a locked position (Figure 8B) where the bulbous portion 350 is prevented from passing through the narrower part of the opening 342, thus locking the cable 345, and therefore the knee, in extension. As a user can simply push and/or pull the locking plate 340 between the locked and unlocked positions, this provides a tool-free way of engaging the locking system. While a sliding plate 340 is shown, it would be apparent this was only one exemplary way of locking the cable 345 in position, and that other ways of selectively engaging the cable, e.g. gripping or otherwise holding, would be suitable for use with the present prosthetic joint. In some cases, the bulbous portion 350 may be omitted. An alternative locking mechanism is shown in Figures 15A and 15B. The locking plate has been replaced by a rotating locking member 380 pivotally attached to the distal part 300 and having a hooked end 385. The locking member 380 can be rotated between a locked position (Figure 15B) and an unlocked (position 15A) to selectively secure the knee joint in the extended position. In the locked position, the hooked end 385 is configured to latch onto an anchoring pin 390 on the proximal part 200 to prevent relative movement between the proximal part 200 and the distal part 300. As the locking member 380 is disposed external to the distal part, a user can easily rotate the locking member 380 using their hand.

Figure 7 illustrates a rear view of the prosthetic knee joint 100. As shown, the distal part 300 has an opening 375 through which an adjustable nut 370 can be accessed and rotated by one or more fingers of a user, for example by mounting the nut on a threaded bar fixed in the pylon 30 (not shown). Rotating the adjustable nut 370 can increase or decrease the initial compression of the compression spring 360, changing the stiffness profile of the knee joint. The opening 375 may be at least partially defined by a shoulder 365 formed at a proximal end of the distal part 300. The adjustable nut 370 provides a tool-free way for the amputee to adjust the stiffness of the compression spring 360, for example, to match their walking speed. By providing a way to adjust the stiffness of the prosthetic knee 10 which does not require disassembly the device, this greatly improves the utility of the device in the field, particularly in remote areas, where specialist support is not typically available.

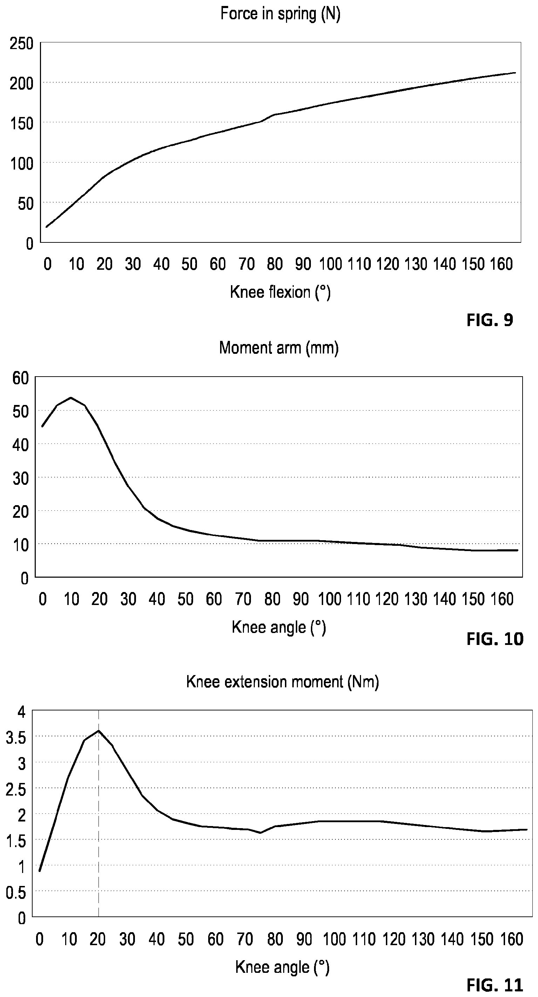

Figure 11 illustrates exemplary torque values using the extensor system of Figures 14A and 14B as the knee flexes through its full range of motion. As explained above, the magnitude of the force vector F will depend on the stiffness and the level of deformation of the compression spring 360 as the knee flexes (see Figure 9). The distal part 300 includes an opening 375 through which the steel cable 345 can extend. Thus, as the knee flexes, the direction of the steel cable 345 relative to the axis of rotation also changes, which will in turn define the moment arm "r" of the force vector F relative to the axis of rotation 430, as shown in Figure 10. The present linkage mechanism 300 is therefore able to provide a bi-phasic extensor moment that can be tuned such that the peak moment is at a pre-determined angle of flexion, while maintaining an extensor moment (i.e. a positive torque) throughout the range of motion of the knee joint. As shown in Figure 11, the presently disclosed arrangement of linkage 410, 420 provides a peak moment at a desired angle of knee flexion, for example between 20 and 40 degrees of knee flexion. Importantly, the level of torque decreases rapidly away from the peak torque, so that the assistance is limited to the desired range. This is desirable, for example, when the amputee is sat on a chair with the knee at 90 degrees and the lower limb needs to hang freely, or when the user is sat, squatting or kneeling with larger angles of knee flexion.

While a prosthetic knee joint has been described above, it would be apparent that the present prosthetic joint would also be suitable for use as a prosthetic elbow joint. In this case, references to the posterior direction should be replaced with the anterior direction and vice versa, to reflect the rotation of the forearm in an anterior direction relative to the upper arm. Similarly, the distal prosthetic limb part would include a forearm and/or a prosthetic hand.

Features, integers, characteristics, or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

## Claims

1. A prosthetic joint (100) comprising:
a proximal part (200) having a proximal side (215), a distal side (220) opposed to the proximal side, and a mounting point (205) on the proximal side (215) configured to connect to a proximal prosthetic limb part (20),
a distal part (300) configured to connect to a distal prosthetic limb part (30), and
a polycentric linkage mechanism (400) connected to the proximal and distal parts (200, 300) and arranged to rotate the distal part (300) from an extended position, where the distal part is disposed on the distal side (220) of the proximal part (200), to a flexed position, where at least a portion of the distal part (300) is disposed on the proximal side (215) of the proximal part (200),
wherein the distal part (300) is arranged to rotate relative to the proximal part (200) about an instantaneous axis of rotation (430), the axis of rotation defining a medial-lateral axis of the proximal part (200),
wherein the linkage mechanism (400) comprises a first linkage (410) and a further pair of linkages (420A, 420B), each of which are pivotally connected to the proximal and distal parts (200, 300) via respective connection points (412A, 412B; 422A, 422B),
wherein the position of the instantaneous axis of rotation (430) is determined by the location of the connection points (412A, 412B; 422A, 422B) of the first linkage (410) and the further pair of linkages (420A, 420B),
**characterised in that** the connection points (412A, 412B) of the first linkage (410) are spaced from a mid-plane of the proximal part (200) by a smaller distance along the medial-lateral axis (ML) than the connection points (422A, 422B) of the pair of linkages (420A, 420B),
**in that** the distal part (300) is spaced from the proximal part (200) in the direction of flexion as the distal part is rotated between the extended position and the flexed position, and
**in that** in the extended position, the connection points (412A, 412B) of the first linkage (410) are spaced from the mounting point (205) by a smaller distance in the direction of flexion than the connection points (422A, 422B) of the pair of linkages (420A, 420B).

2. A prosthetic joint according to claim 1, wherein, in the extended position, the first linkage (410) forms a first angle (B) relative to a longitudinal axis (SI) of the proximal part (200) and the pair of linkages (420A, 420B) form a second angle (A) relative to the longitudinal axis (SI) of the proximal part (200), and wherein the second angle (A) is smaller than the first angle (B).

3. A prosthetic joint according to claim 2, wherein the first angle (B) is between 5 and 30 degrees and/or the second angle (A) is between 0 and 15 degrees.

4. A prosthetic joint according to any preceding claim, wherein, in the extended position, the first linkage (410) and the pair of linkages (420A, 420B) are oriented in the direction of flexion.

5. A prosthetic joint according to any preceding claim, wherein the connection points (412A, 412B; 422A, 422B) of the first linkage (410) and the pair of linkages (420A, 420B) are disposed on the same side of the mounting point (205).

6. A prosthetic joint according to any preceding claim, wherein the distal part (300) comprises a channel (310) for receiving a portion of the distal prosthetic limb part (30), and a clamp (315) for releasably securing the portion of the distal prosthetic limb part in the channel, optionally wherein the clamp (315) comprises a body formed as an open-ended loop with a pair of ends (330) spaced from one another, a pin extending through the pair of ends and fixed to one of the pair of ends, and a lever (320) rotatably connected to the pin, wherein the lever is eccentrically mounted to the pin to provide a camming surface (325) on the lever, wherein, when rotated to a closed position, the camming surface (325) is arranged to abut one of the ends of the body so as to deform the body to clamp the distal part (300) to the distal prosthetic limb part (30), and wherein, when rotated to an open position, the camming surface (325) is arranged to release the body from the distal part (300) so as to allow the distal prosthetic limb part (30) to move relative to the distal part (300).

7. A prosthetic joint according to any preceding claim, further comprising an extensor system configured to apply a torque to rotate the distal part (300) to the extended position, optionally wherein the extensor system is arranged to apply a peak torque when the distal part (300) is rotated between 20 and 40 degrees from the extended position.

8. A prosthetic joint according to claim 7, wherein the extensor system is biased to rotate the distal part (300) to the extended position across a range of motion of the linkage mechanism.

9. A prosthetic joint according to claim 7 or claim 8, wherein the extensor system comprises a tensile element (345) connected to a resiliently deformable element (360) and the proximal part (200), and wherein, as the distal part (300) rotates from the extended position to the flexed position, the resiliently deformable element (360) is arranged to deform so as to exert the torque, optionally wherein the resiliently deformable member (360) is disposed within a tubular element of the distal part (300), and wherein at least a portion of the tensile element extends through the tubular element.

10. A prosthetic joint according to claim 9, wherein the extensor system comprises a locking member (340) arranged to selectively lock the tensile element (345) to prevent further rotation of the distal part (300) towards the flexed position, optionally wherein the locking member (340) is operable by a hand of a user.

11. A prosthetic joint according to any of claims 7 to 10, wherein the torque of the extensor system is adjustable by a hand of a user.

12. A prosthetic joint according to any preceding claim, wherein the linkage mechanism (400) is arranged to provide a range of motion of at least 160 degrees between the extended position and the flexed position.

13. A prosthetic joint according to any preceding claim, wherein the proximal part (200) comprises a proximal end (215) and a distal end (220), and wherein the distance (225) between the proximal and distal ends is as little as 40mm.

14. A prosthetic joint according to any preceding claim, wherein the distal part (200) comprises a support (395) for supporting the proximal prosthetic limb part (20) when the joint (100) is rotated to maximum flexion, so as to provide a load path between the proximal prosthetic limb part (20) and an external surface via the distal part (300).

15. A prosthetic limb (10) comprising:
a prosthetic joint (100) according to any preceding claim,
a proximal prosthetic limb part (20) connected to the proximal part (200) of the prosthetic joint, and
a distal prosthetic limb part (30) connected to the distal part (300) of the prosthetic joint.

## Patentansprüche

1. Prothesengelenk (100), umfassend:
einen proximalen Teil (200), der eine proximale Seite (215), eine distale Seite (220) gegenüber der proximalen Seite und einen Montagepunkt (205) an der proximalen Seite (215), der konfiguriert ist, um mit einem proximalen Prothesengliedteil (20) verbunden zu werden,
einen distalen Teil (300), der konfiguriert ist, um mit einem distalen Prothesengliedteil (30) verbunden zu werden, und
einen polyzentrischen Kopplungsmechanismus (400), der mit dem proximalen und dem distalen Teil (200, 300) verbunden und angeordnet ist, um den distalen Teil (300) aus einer gestreckten Position, in der der distale Teil auf der distalen Seite (220) des proximalen Teils (200) angeordnet ist, in eine gebeugte Position zu drehen, in der zumindest ein Abschnitt des distalen Teils (300) auf der proximalen Seite (215) des proximalen Teils (200) angeordnet ist,
wobei der distale Teil (300) angeordnet ist, um in Bezug auf den proximalen Teil (200) um eine momentane Drehachse (430) zu drehen, wobei die Drehachse eine medial-laterale Achse des proximalen Teils (200) definiert,
wobei der Kopplungsmechanismus (400) eine erste Kopplung (410) und ein weiteres Kopplungspaar (420A, 420B) umfasst, wovon jede über jeweilige Verbindungspunkte (412A, 412B; 422A, 422B) schwenkbar mit dem proximalen und dem distalen Teil (200, 300) verbunden ist,
wobei die Position der momentanen Drehachse (430) durch die Stelle der Verbindungspunkte (412A, 412B; 422A, 422B) der ersten Kopplung (410) und des weiteren Kopplungspaars (420A, 420B) bestimmt wird,
**dadurch gekennzeichnet, dass**
die Verbindungspunkte (412A, 412B) der ersten Kopplung (410) entlang der mediallateralen Achse (ML) um einen geringeren Abstand von einer mittleren Ebene des proximalen Teils (200) beabstandet sind als die Verbindungspunkte (422A, 422B) des Kopplungspaars (420A, 420B),
das der distale Teil (300) in Beugungsrichtung von dem proximalen Teil (200) beabstandet ist, wenn der distale Teil zwischen der gestreckten Position und der gebeugten Position gedreht wird, und,
dass die Verbindungspunkte (412A, 412B) der ersten Kopplung (410) in der gestreckten Position in Biegerichtung um einen geringeren Abstand zu dem Montagepunkt (205) beabstandet sind als die Verbindungspunkte (422A, 422B) des Kopplungspaars (420A, 420B).

2. Prothesengelenk nach Anspruch 1, wobei in der gestreckten Position die erste Kopplung (410) einen ersten Winkel (B) in Bezug auf eine Längsachse (Sl) des proximalen Teils (200) bildet und das Kopplungspaar (420A, 420B) einen zweiten Winkel (A) in Bezug auf die Längsachse (Sl) des proximalen Teils (200) bildet, und wobei der zweite Winkel (A) kleiner ist als der erste Winkel (B).

3. Prothesengelenk nach Anspruch 2, wobei der erste Winkel (B) zwischen 5 und 30 Grad ist und/oder der zweite Winkel (A) zwischen 0 und 15 Grad ist.

4. Prothesengelenk nach einem vorstehenden Anspruch, wobei die erste Kopplung (410) und das Kopplungspaar (420A, 420B) in der gestreckten Position in Biegerichtung ausgerichtet sind.

5. Prothesengelenk nach einem vorstehenden Anspruch, wobei die Verbindungspunkte (412A, 412B; 422A, 422B) der ersten Kopplung (410) und des Kopplungspaars (420A, 420B) auf derselben Seite des Montagepunkts (205) angeordnet sind.

6. Prothesengelenk nach einem vorstehenden Anspruch, wobei der distale Teil (300) einen Kanal (310) zum Aufnehmen eines Abschnitts des distalen Prothesengliedteils (30) und eine Klemme (315) zum lösbaren Fixieren des Abschnitts des distalen Prothesengliedteils in dem Kanal umfasst, wobei die Klemme (315) optional einen Körper, der als offene Schlaufe mit einem Paar voneinander beabstandeter Enden (330) gebildet ist, einen Stift, der sich durch das Paar von Enden erstreckt und an einem der zwei Enden befestigt ist, und einen Hebel (320), der drehbar mit dem Stift verbunden ist, umfasst, wobei der Hebel exzentrisch an dem Stift montiert ist, um eine Nockenfläche (325) an dem Hebel bereitzustellen, wobei die Nockenfläche (325), wenn sie in eine geschlossene Position gedreht ist, angeordnet ist, um an einem der Enden des Körpers anzuliegen, um den Körper zu verformen, um den distalen Teil (300) an dem distalen Prothesengliedteil (30) festzuklemmen, und wobei die Nockenfläche (325), wenn sie in eine offene Position gedreht ist, angeordnet ist, um den Körper von dem distalen Teil (300) freizugeben, um zu ermöglichen, dass sich der distale Prothesengliedteil (30) in Bezug auf den distalen Teil (300) bewegt.

7. Prothesengelenk nach einem vorstehenden Anspruch, ferner umfassend ein Strecksystem, das konfiguriert ist, um ein Drehmoment auszuüben, um den distalen Teil (300) in die gestreckte Position zu drehen, optional wobei das Strecksystem angeordnet ist, um ein Spitzendrehmoment auszuüben, wenn der distale Teil (300) aus der gestreckten Position um 20 bis 40 Grad gedreht wird.

8. Prothesengelenk nach Anspruch 7, wobei das Strecksystem vorgespannt ist, um den distalen Teil (300) über einen Bewegungsbereich des Kopplungsmechanismus in die gestreckte Position zu drehen.

9. Prothesengelenk nach Anspruch 7 oder Anspruch 8, wobei das Strecksystem ein Zugelement (345) umfasst, das mit einem elastisch verformbaren Element (360) und dem proximalen Teil (200) verbunden ist, und wobei das elastisch verformbare Element (360) beim Drehen des distalen Teils (300) aus der gestreckten Position in die gebeugte Position angeordnet ist, um zu verformen, um das Drehmoment auszuüben, optional wobei das elastisch verformbare Element (360) innerhalb eines rohrförmigen Elements des distalen Teils (300) angeordnet ist, und wobei sich zumindest ein Abschnitt des Zugelements durch das rohrförmige Element erstreckt.

10. Prothesengelenk nach Anspruch 9, wobei das Strecksystem ein Verriegelungsorgan (340) umfasst, das angeordnet ist, um das Zugelement (345) selektiv zu verriegeln, um eine weitere Drehung des distalen Teils (300) in Richtung der gebeugten Position zu verhindern, optional wobei das Verriegelungselement (340) durch eine Hand eines Benutzers betätigbar ist.

11. Prothesengelenk nach einem der Ansprüche 7 bis 10, wobei das Drehmoment des Strecksystems durch eine Hand eines Benutzers einstellbar ist.

12. Prothesengelenk nach einem vorstehenden Anspruch, wobei der Kopplungsmechanismus (400) angeordnet ist, um einen Bewegungsbereich von mindestens 160 Grad zwischen der gestreckten und der gebeugten Position bereitzustellen.

13. Prothesengelenk nach einem vorstehenden Anspruch, wobei der proximale Teil (200) ein proximales Ende (215) und ein distales Ende (220) umfasst, und wobei der Abstand (225) zwischen dem proximalen und dem distalen Ende nur 40 mm ist.

14. Prothesengelenk nach einem vorstehenden Anspruch, wobei der distale Teil (200) eine Halterung (395) zum Halten des proximalen Prothesengliedteils (20) umfasst, wenn das Gelenk (100) bis zur maximalen Beugung gedreht wird, um über den distalen Teil (300) einen Lastpfad zwischen dem proximalen Prothesengliedteil (20) und einer Außenfläche bereitzustellen.

15. Prothesenglied (10), umfassend:
ein Prothesengelenk (100) nach einem vorstehenden Anspruch,
einen proximalen Prothesengliedteil (20), der mit dem proximalen Teil (200) des Prothesengelenks verbunden ist, und
einen distalen Prothesengliedteil (30), der mit dem distalen Teil (300) des Prothesengelenks verbunden ist.

## Revendications

1. Articulation prothétique (100) comprenant :
une partie proximale (200) comportant un côté proximal (215), un côté distal (220) opposé au côté proximal, et un point de montage (205) sur le côté proximal (215) conçu pour être relié à une partie de membre prothétique proximale (20),
une partie distale (300) conçue pour être reliée à une partie de membre prothétique distale (30), et
un mécanisme de liaison polycentrique (400) relié aux parties proximale et distale (200, 300) et agencé pour faire tourner la partie distale (300) d'une position étendue, dans laquelle la partie distale est disposée sur le côté distal (220) de la partie proximale (200), à une position fléchie, dans laquelle au moins une portion de la partie distale (300) est disposée sur le côté proximal (215) de la partie proximale (200),
dans laquelle la partie distale (300) est agencée pour tourner par rapport à la partie proximale (200) autour d'un axe instantané de rotation (430), l'axe de rotation définissant un axe médio-latéral de la partie proximale (200),
dans laquelle le mécanisme de tringlerie (400) comprend une première biellette (410) et une paire de biellettes (420A, 420B) supplémentaire, dont chacune est reliée de manière pivotante aux parties proximale et distale (200, 300) par l'intermédiaire de points de liaison (412A, 412B ; 422A, 422B) respectifs,
dans laquelle la position de l'axe instantané de rotation (430) est déterminée par l'emplacement des points de liaison (412A, 412B ; 422A, 422B) de la première biellette (410) et de la paire de biellettes (420A, 420B) supplémentaire,
**caractérisée en ce que**
les points de liaison (412A, 412B) de la première biellette (410) sont espacés d'un plan médian de la partie proximale (200), d'une distance plus petite le long de l'axe médio-latéral (ML) que les points de liaison (422A, 422B) de la paire de biellettes (420A, 420B),
**en ce que** la partie distale (300) est espacée de la partie proximale (200) dans la direction de flexion à mesure que la partie distale est tournée entre la position étendue et la position fléchie, et
**en ce que**, dans la position étendue, les points de liaison (412A, 412B) de la première biellette (410) sont espacés du point de montage (205) d'une distance plus petite dans la direction de flexion que les points de liaison (422A, 422B) de la paire de biellettes (420A, 420B).

2. Articulation prothétique selon la revendication 1, dans laquelle, dans la position étendue, la première biellette (410) forme un premier angle (B) par rapport à un axe longitudinal (SI) de la partie proximale (200) et la paire de biellettes (420A, 420B) forme un second angle (A) par rapport à l'axe longitudinal (SI) de la partie proximale (200), et dans laquelle le second angle (A) est inférieur au premier angle (B).

3. Articulation prothétique selon la revendication 2, dans laquelle le premier angle (B) est compris entre 5 et 30 degrés et/ou le second angle (A) est compris entre 0 et 15 degrés.

4. Articulation prothétique selon une quelconque revendication précédente, dans laquelle, dans la position étendue, la première biellette (410) et la paire de biellettes (420A, 420B) sont orientées dans la direction de flexion.

5. Articulation prothétique selon une quelconque revendication précédente, dans laquelle les points de liaison (412A, 412B ; 422A, 422B) de la première biellette (410) et de la paire de biellettes (420A, 420B) sont disposés sur le même côté du point de montage (205).

6. Articulation prothétique selon une quelconque revendication précédente, dans laquelle la partie distale (300) comprend un canal (310) pour recevoir une portion de la partie de membre prothétique distale (30), et un dispositif de serrage (315) pour fixer de manière libérable la portion de la partie de membre prothétique distale dans le canal, éventuellement dans laquelle le dispositif de serrage (315) comprend un corps formé sous la forme d'une boucle à extrémité ouverte avec une paire d'extrémités (330) espacées l'une de l'autre, une broche s'étendant à travers la paire d'extrémités et fixée à l'une de la paire d'extrémités, et un levier (320) relié de manière rotative à la broche, dans laquelle le levier est monté de manière excentrique sur la broche pour fournir une surface de came (325) sur le levier, dans laquelle, lorsqu'il est tourné vers une position fermée, la surface de came (325) est agencée pour venir en butée contre l'une des extrémités du corps de manière à déformer le corps pour serrer la partie distale (300) à la partie de membre prothétique distale (30), et dans laquelle, lorsqu'il est tourné vers une position ouverte, la surface de came (325) est agencée pour libérer le corps de la partie distale (300) de manière à permettre à la partie de membre prothétique distale (30) de se déplacer par rapport à la partie distale (300).

7. Articulation prothétique selon une quelconque revendication précédente, comprenant en outre un système extenseur conçu pour appliquer un couple pour faire tourner la partie distale (300) vers la position étendue, éventuellement dans laquelle le système extenseur est agencé pour appliquer un couple maximal lorsque la partie distale (300) est tournée entre 20 et 40 degrés par rapport à la position étendue.

8. Articulation prothétique selon la revendication 7, dans laquelle le système extenseur est sollicité pour faire tourner la partie distale (300) vers la position étendue sur une plage de mouvement du mécanisme de tringlerie.

9. Articulation prothétique selon la revendication 7 ou la revendication 8, dans laquelle le système extenseur comprend un élément de traction (345) relié à un élément déformable élastiquement (360) et à la partie proximale (200), et dans laquelle, à mesure que la partie distale (300) tourne de la position étendue à la position fléchie, l'élément déformable élastiquement (360) est agencé pour se déformer de manière à exercer le couple, éventuellement dans laquelle l'élément déformable élastiquement (360) est disposé à l'intérieur d'un élément tubulaire de la partie distale (300), et dans laquelle au moins une portion de l'élément de traction s'étend à travers l'élément tubulaire.

10. Articulation prothétique selon la revendication 9, dans laquelle le système extenseur comprend un élément de verrouillage (340) agencé pour verrouiller sélectivement l'élément de traction (345) afin d'empêcher une rotation supplémentaire de la partie distale (300) vers la position fléchie, éventuellement dans laquelle l'élément de verrouillage (340) peut être actionné par une main d'un utilisateur.

11. Articulation prothétique selon l'une quelconque des revendications 7 à 10, dans laquelle le couple du système extenseur est réglable par une main d'un utilisateur.

12. Articulation prothétique selon une quelconque revendication précédente, dans laquelle le mécanisme de tringlerie (400) est agencé pour fournir une plage de mouvement d'au moins 160 degrés entre la position étendue et la position fléchie.

13. Articulation prothétique selon une quelconque revendication précédente, dans laquelle la partie proximale (200) comprend une extrémité proximale (215) et une extrémité distale (220), et dans laquelle la distance (225) entre les extrémités proximale et distale est aussi faible que 40 mm.

14. Articulation prothétique selon une quelconque revendication précédente, dans laquelle la partie distale (200) comprend un support (395) pour supporter la partie de membre prothétique proximale (20) lorsque l'articulation (100) est tournée jusqu'à une flexion maximale, de manière à fournir un chemin de charge entre la pièce de membre prothétique proximale (20) et une surface externe par l'intermédiaire de la partie distale (300).

15. Membre prothétique (10) comprenant :
une articulation prothétique (100) selon une quelconque revendication précédente,
une partie de membre prothétique proximale (20) reliée à la partie proximale (200) de l'articulation prothétique, et
une partie de membre prothétique distale (30) reliée à la partie distale (300) de l'articulation prothétique.
